# FASCICULE DE BREVET EUROPEEN

(11) **EP 2 666 441 B1**
(45) Date de publication et mention de la délivrance du brevet: **05.08.2015**
(21) Numéro de dépôt: 13162455.3
(22) Date de dépôt: 05.04.2013
(51) Int. Cl.: A61F 2/00

(54) **Dispositif d'attache implantable, implant pour le traitement des prolapsus de la statique pelvienne comportant un tel dispositif et kit comprenant ledit dispositif**
Implantierbare Befestigungsvorrichtung, Implantat zur Behandlung eines Beckenbodenprolaps, das eine solche Vorrichtung umfasst, und Kit, das eine solche Vorrichtung umfasst
Implantable fastener device, implant for the treatment of pelvic floor prolapse comprising such a device and kit including said device

(30) Priorité: 22.05.2012 FR 1254648
(43) Date de publication de la demande: 27.11.2013
(73) Titulaire: Cousin Biotech, 59117 Wervicq Sud (FR)
(72) Inventeur: Noel, Stéphane, 59496 HANTAY (FR); Kamche, Farid, 59200 TOURCOING (FR); Solecki, Gilles, 59390 LANNOY (FR)
(74) Mandataire: Balesta, Pierre

(56) Documents cités:
- EP-A1- 1 399 088
- WO-A1-2007/014241
- WO-A2-2009/102945
- US-A1- 2005 131 393
- US-A1- 2010 016 894

## Description

La présente invention concerne le domaine technique des dispositifs d'attache implantables solidarisés ou aptes à être solidarisés, d'une part, à un implant et, d'autre part, à un outil chirurgical, tel qu'un ancillaire.

Le prolapsus génital est une migration anormale d'un ou plusieurs organes pelviens modifiant la forme des parois vaginales pouvant aller jusqu'à leur extériorisation à travers la fente uro-génitale. La vessie, l'utérus, le rectum sont les principaux organes concernés par cette extériorisation. On parle respectivement pour ces organes de cystocèle, d'hystérocèle et de rectocèle. Le prolapsus peut également concerner le dôme vaginal en présence d'hystérectomie, le cul de sac de Douglas seul (élytrocèle) ou associé à des anses intestinales (entérocèle).

Une grande variété de techniques chirurgicales existe pour corriger les prolapsus vaginaux.

Les approches chirurgicales sont assez variées et consistent à placer une structure textile au niveau du plancher pelvien. Le placement de cette structure textile peut se faire par voie vaginale, abdominale ou par laparoscopie.

Une première technique est la sacro-colpopexie par voie abdominale invasive. Elle consiste à suspendre le col de l'utérus, le dôme vaginal, ou l'isthme utérin, au ligament longitudinal antérieur en avant du promontoire, à la jonction des vertèbres L5-S1, par l'intermédiaire de deux prothèses antérieure et postérieure. Elle peut être associée à des gestes chirurgicaux complémentaires comme l'hystérectomie ou une douglassectomie par exemple.

La seconde technique dite « sans tension » consiste à interposer une prothèse tricotée à base de fils monofilamentaires en polypropylène entre la vessie et le vagin pour une cystocèle ou entre le rectum et le vagin dans le cas d'une rectocèle.

Le traitement chirurgical a pour but de remplacer les moyens de suspension (fascias, ligaments) ou les moyens de soutènement (muscles du périnée) devenus défaillants et le plus souvent les deux. Il fait appel, de nos jours, à l'utilisation de « prothèses » synthétiques tricotées. Ces prothèses servent à remplacer les fascias défaillants, ou à suspendre les organes « descendus » à des ligaments naturels solides. Ce traitement peut se faire selon trois voies chirurgicales différentes :
- en ouvrant l'abdomen (« laparotomie ») ;
- par coelioscopie (« laparoscopie ») ;
- en passant par le vagin (« voie vaginale »).

L'efficacité des trois voies chirurgicales, dans les mains d'un chirurgien expérimenté, est la même. La simplicité technique, le moindre taux de complications, et la durée d'intervention plus courte font préférer, pour beaucoup d'écoles chirurgicales, la voie vaginale chez les femmes en ménopause ou en pré-ménopause. La coelioscopie ou la laparotomie sont plus souvent réservées aux femmes plus jeunes, en raison de la meilleure efficacité à long terme et de la meilleure résistance aux importants efforts physiques.

La technique par voie vaginale trans-obturatrice est décrite dans les brevets WO 2007/016698 et WO 2005/122721. Elle nécessite l'utilisation de prothèse de forme plane munie de bras. La partie plane est interposée entre l'organe prolabé et le vagin tandis que les bras de la prothèse traversent les trous obturateurs du bassin. Pour placer la prothèse dans cette configuration, le chirurgien utilise des aiguilles courbes ou hélicoïdales, appelées communément ancillaires. Ces aiguilles perforent les trous obturateurs de l'extérieur du corps vers l'intérieur en traversant la peau pour ressortir dans la partie antérieure du vagin et déterminent ainsi le passage selon lequel l'implant doit être disposé. Les bras de la prothèse sont ensuite fixés sur la pointe de l'aiguille par un moyen de fixation L'aiguille est ensuite passée en sens inverse de son passage d'introduction pour placer les bras dans les trous obturateurs du bassin, puis est retirée. Le moyen de fixation est également retiré en sorte que seul l'implant reste dans l'organisme.

Le document EP 1.399.088 B1 décrit ainsi succinctement aux figures 7f et 7g un moyen de fixation entre l'aiguille de l'ancillaire et un implant pour le traitement des prolapsus de la statique pelvienne. Ce moyen de fixation peut être un noeud simple ou double formant une boucle d'attache apte à coopérer avec une rainure ménagée sur l'extrémité distale de l'aiguille de l'ancillaire, et solidarisé d'autre part avec l'implant. Cette boucle d'attache ne permet pas d'assurer une liaison fiable et durable entre l'aiguille et l'implant. La boucle est simplement serrée manuellement par le chirurgien, il n'est ainsi pas possible de maîtriser l'effort de serrage de la boucle sur l'extrémité distale de l'aiguille de l'ancillaire. Cet effort de serrage est ainsi dépendant du chirurgien et donc aléatoire et peu reproductible. Par ailleurs, même si le chirurgien applique un effort de serrage suffisant sur la boucle, il a été observé que ce moyen de fixation se relaxera par glissement des fils de la boucle pendant le passage de l'aiguille dans les trous obturateurs ou les tissus. Il a en effet été observé que la boucle formée se relaxe pendant le retrait de l'aiguille et provoque la perte de l'implant destiné à être placé, ce qui est rédhibitoire dans cette technique consistant à passer à l'aveugle dans les tissus ou trous trans-obturateurs l'implant en tension avec le moyen de fixation solidarisé sur l'extrémité distale de l'aiguille.

La présente invention a ainsi pour objet un dispositif d'attache implantable pour la liaison fiable, reproductible et durable d'un implant pour le traitement des prolapsus de la statique pelvienne avec l'aiguille d'un ancillaire.

La présente invention a également pour objet un dispositif d'attache simple à mettre en oeuvre, peu onéreux et ayant un encombrement réduit pour son passage dans les tissus et les trous trans-obturateurs.

La présente invention a ainsi pour objet, selon un premier aspect, un dispositif d'attache implantable comprenant un élément longiligne tubulaire souple et creux délimitant un volume intérieur comportant une boucle de serrage, de circonférence (P) en position inactive, formée par la disposition d'une portion dudit élément tubulaire dans ledit volume intérieur entre des orifices d'admission et de sortie distant d'une longueur (I1), ladite portion ayant des extrémités avant et arrière. Le dispositif comprend également des moyens de blocage de ladite portion dans le volume intérieur dudit élément longiligne, lesdits moyens de blocage étant désactivés sous l'effet d'une traction manuelle exercée sur la première ou seconde extrémité dudit élément longiligne, ladite traction permettant le coulissement dudit élément longiligne entre lesdits orifices et corrélativement la formation d'une boucle de serrage de circonférence (p) en position active, (p) étant inférieur à (P).

On comprend par élément longiligne tubulaire, un élément longiligne souple apte à former une boucle sur lui-même, de préférence un élément textile creux, tel qu'un tricot tubulaire ou une tresse tubulaire.

De préférence, les extrémités avant et arrière de ladite portion sont disposées respectivement en amont de l'orifice d'admission et en aval de l'orifice de sortie.

En fonctionnement, le chirurgien dispose la boucle de serrage dans sa position inactive sur l'extrémité distale d'une aiguille d'un ancillaire, puis exerce une traction sur la première ou la seconde extrémité dudit élément en sorte de désactiver les moyens de blocage et serrer en force la boucle de serrage sur ladite extrémité distale de l'aiguille, la boucle adoptant alors une circonférence (p) en position active inférieure à la circonférence initiale (P) dans sa position inactive. La désactivation des moyens de blocage est effectuée sous l'effet d'une contrainte manuelle de force déterminée (daN) en moins de quelques secondes, permettant ainsi un serrage uniforme du dispositif d'attache sur l'extrémité distale de l'aiguille.

La demanderesse a également observé que le coulissement d'une portion dudit élément longiligne dans son volume intérieur jusqu'à la formation d'une boucle de serrage de circonférence (p) permet d'assurer un serrage instantané et durable en sorte que l'implant puisse être passé et guidé par l'ancillaire via le dispositif d'attache selon l'invention.

Bien sûr, la force de déblocage (daN) des moyens de blocage est déterminée en sorte de ne pas être trop élevée et d'éviter d'exercer des tensions sur les tissus ou les trous obturateurs puisque la traction exercée sur l'une des extrémités dudit élément longiligne est effectuée lorsque l'aiguille de l'ancillaire est passée dans les tissus et les trous trans-obturateurs.

Le passage de la boucle de serrage d'une position inactive à une position active est quasi-instantanée, ce qui garantit la fiabilité et la durée de la solidarisation du dispositif d'attache à l'extrémité distale de l'aiguille de l'ancillaire.

Dans une variante, les moyens de blocage comprennent un revêtement dans au moins un polymère appliqué sur la surface extérieure et le pourtour de l'élément longiligne sur la longueur (l1) séparant les orifices d'admission et de sortie.

La rupture du revêtement engendrée par la traction exercée par le chirurgien sur la première ou seconde extrémité dudit élément longiligne permet de faire passer la boucle de serrage d'une position inactive à une position active de façon quasi-instantanée.

Dans une variante, la masse du revêtement est inférieure ou égale à 20 g/mètre dudit élément tubulaire souple.

Il n'est en effet pas nécessaire de disposer une quantité de revêtement trop importante entre les orifices d'admission et de sortie, le but étant simplement d'empêcher le coulissement naturel de ladite portion dudit élément dans son volume intérieur et d'engendrer une certaine résistance obligeant le chirurgien à exercer une contrainte à peine de quelques kilos pour serrer la boucle de serrage sur l'extrémité distale de l'aiguille de l'ancillaire.

Dans une variante, les moyens de blocage comprennent au moins une première sphère dans au moins un polymère obturant l'orifice d'admission, ladite sphère étant disposée autour de l'extrémité avant de ladite portion, et agencée en sorte que la traction manuelle exercée sur la première ou la seconde extrémité dudit élément longiligne entraîne le passage en force de la dite première sphère dans le volume intérieur dudit élément longiligne jusqu'à sa sortie par ledit orifice de sortie.

Dans une variante, les moyens de blocage comprennent une seconde sphère dans au moins un polymère obturant l'orifice de sortie, ladite sphère étant disposée autour de l'extrémité arrière de ladite portion.

Dans une variante, ledit matériau polymère est choisi seul ou en combinaison parmi les polymères suivants : polydiméthysiloxane (PDMS), polyuréthane, polymère d'acide lactique de forme L ou D (PLLA ou PLDA), polyamide (PA 66 notamment).

Les matériaux polymères formant la ou lesdites sphères et/ou ledit revêtement peuvent être de la même nature.

Dans une variante, la boucle de serrage présente une circonférence (P) en position inactive inférieure à 5 cm, de préférence inférieure à 3 cm.

La boucle de serrage présente une circonférence supérieure à 0 cm, de préférence supérieure à 0,5 cm.

La demanderesse a observé que le coulissement dudit élément longiligne sur lui-même ne devrait pas être effectué sur une trop grande distance afin d'obtenir un serrage quasi-instantanée et donc fiable.

Dans une variante, la longueur (l1) séparant les orifices d'admission et de sortie est inférieure ou égale à 3 cm, de préférence inférieure ou égale à 1 cm. La longueur (I1) est supérieure à 0 cm.

Cette disposition contribue également à assurer un serrage quasi-instantané et fiable.

Dans une variante, l'élément longiligne est un élément textile, en particulier une tresse ou un tricot, comprenant un ou plusieurs fil(s) multifilamentaire(s) et/ou un ou plusieurs fil(s) monofilamentaire(s).

La présence de fils permet d'engendrer davantage de frottement lors du coulissement dudit élément longiligne sur lui-même et par là même d'améliorer la fiabilité et la tenue dans le temps de la boucle de serrage en position active sur l'extrémité distale de l'aiguille de l'ancillaire.

Dans une variante, l'élément longiligne a une masse linéique inférieure ou égale à 25 g/mètre, de préférence inférieure ou égale à 15 g/mètre, et encore de préférence inférieure ou égale à 2 g/mètre.

L'élément longiligne selon l'invention est très léger et donc peu invasif.

Dans une variante, la traction exercée sur la première ou la seconde extrémité permettant de désactiver les moyens de blocage est inférieure ou égale à 4 daN, de préférence inférieure ou égale à 2,5 daN.

De préférence, la traction exercée sur la première ou la seconde extrémité est supérieure à 0,5 daN.

La demanderesse a déterminé que ces intervalles de forces de serrage permettent d'obtenir un serrage quasi-instantané sans exercer de tension sur l'aiguille et donc sur les tissus ou les trous trans-obturateurs dans lesquels est disposée ladite aiguille.

La présente invention a pour objet, selon un deuxième aspect, un implant pour le traitement des prolapsus de la statique pelvienne, notamment pour la cure de l'hystérocèle, de la cystocèle ou de la rectocèle, configuré en sorte de présenter une partie centrale supportante et au moins deux parties latérales d'ancrage disposées de part et d'autre de ladite partie centrale comprenant un dispositif d'attache selon l'une des quelconque des variantes de réalisation décrites ci-dessus. Avantageusement, la seconde extrémité dudit élément longiligne est solidarisée ou apte à être solidarisée à l'une ou aux deux parties latérale d'ancrage.

De préférence, la seconde extrémité dudit élément longiligne est solidarisée à au moins une partie latérale d'ancrage par une couture, une épissure ou par l'intermédiaire d'un ou plusieurs noeud(s) entre ladite extrémité et ladite partie latérale.

La présente invention a pour objet, selon un troisième aspect, un kit comprenant un dispositif d'attache selon l'une quelconque des variantes de réalisation précédentes, et un ancillaire.

Ledit ancillaire comprend une aiguille pourvue selon son extrémité distale d'une zone d'attache apte à coopérer avec la boucle de serrage dudit dispositif d'attache.

De préférence, le kit selon l'invention comprend également un implant pour le traitement des prolapsus de la statique pelvienne selon l'une des variantes de réalisation décrites ci-dessus.

Dans une variante, la zone d'attache présente une longueur (12) et comporte des butées avant et arrière aptes à bloquer dans ladite zone d'attache la boucle de serrage dudit élément longiligne lorsque la boucle de serrage est en position active.

De préférence, la longueur (12) est supérieure ou égale à 3 mm, encore de préférence supérieure ou égale à 5 mm, encore de préférence inférieure ou égale à 20 mm.

De préférence, la zone d'attache est de forme cylindrique et présente une circonférence inférieure ou égale à la circonférence de la boucle d'attache dans sa position active (p). En effet, la forme cylindrique de la zone d'attache évite la présence d'arête ou de surface pouvant être contondante pour ledit élément longiligne.

La présente invention sera mieux comprise à la lecture de la description des deux exemples de réalisation suivants cités à titre non limitatif, et illustrés par les figures suivantes, annexées à la présente :
- la figure 1 est une représentation schématique et en perspective d'un premier exemple de réalisation de dispositif d'attache selon l'invention selon une première variante,
- la figure 2 est une représentation schématique et en perspective d'une seconde variante du dispositif d'attache représenté à la figure 1,
- la figure 3 est une représentation schématique et en perspective d'un second exemple de dispositif d'attache selon l'invention selon une première variante,
- la figure 4 est une représentation schématique et en perspective d'une seconde variante du dispositif d'attache représenté à la figure 3,
- la figure 5 est une représentation schématique et en perspective d'un kit selon l'invention comprenant un ancillaire et le premier exemple de dispositif d'attache représenté à la figure 1 en position inactive,
- la figure 6 est une représentation schématique et en perspective du kit représenté à la figure 5 dans lequel le dispositif d'attache est en position active,
- la figure 7 représente une méthode de mesure de la force nécessaire pour désactiver les moyens de blocage selon l'invention.

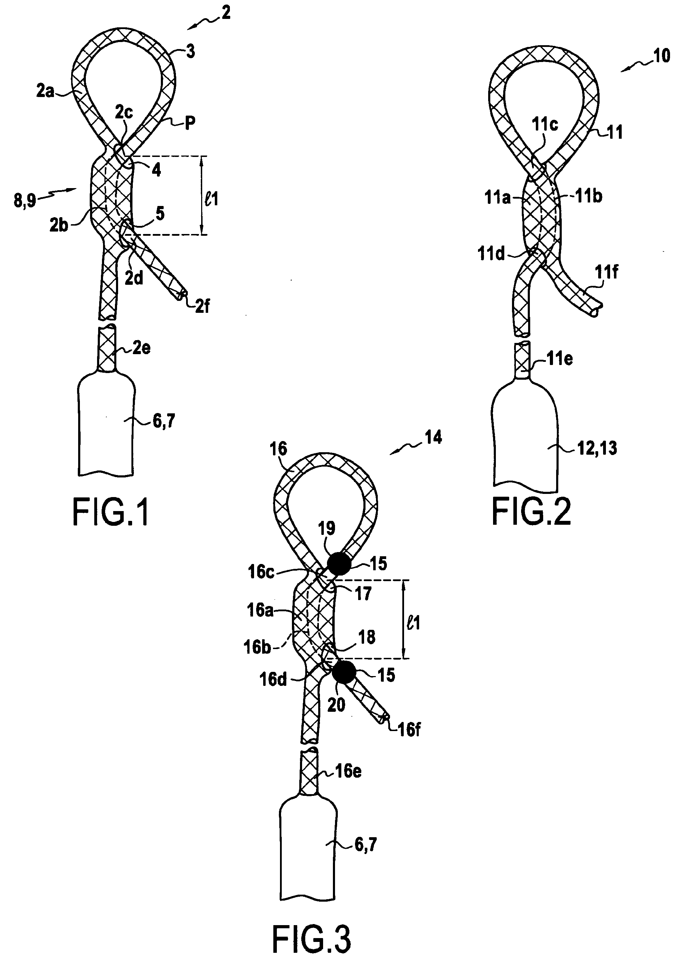

La première variante du premier exemple de dispositif d'attache 1 représenté à la figure 1 comprend un élément longiligne tubulaire souple et creux 2 délimitant un volume intérieur 2a comportant une boucle de serrage 3, de circonférence (P) en position inactive, formée par la disposition d'une portion 2b dudit élément tubulaire 2 dans ledit volume intérieur 2a entre des orifices d'admission 4 et de sortie 5 distants d'une longueur (I1), ladite portion 2b ayant des extrémités avant 2c et arrière 2d.

De préférence, la longueur (l1) séparant les orifices d'admission 4 et de sortie 5 est inférieure ou égale à 3 cm, de préférence inférieure ou égale à 1 cm.

Le dispositif d'attache 1 comprend une boucle de serrage 3 en position inactive ayant une circonférence (P) inférieure ou égale à 5 cm, de préférence inférieure ou égale à 3 cm.

Ledit élément longiligne 2 est pourvu de première 2f et seconde 2e extrémités. Dans cet exemple, la première extrémité 2f est introduite dans le volume intérieur 2a de l'élément longiligne 2 par l'orifice d'admission 4 et en ressort par l'orifice de sortie 5 formant ainsi une portion 2b dudit élément longiligne 2 logée dans son volume intérieur 2a. La première extrémité 2f est dans cet exemple précis libre et peut être saisie par le chirurgien pour provoquer le coulissement de la portion 2b dans le volume intérieur 2a de l'élément longiligne 2. La seconde extrémité 2e de l'élément longiligne 2 est ici représentée solidarisée à une partie latérale d'ancrage 6 d'un implant 7 selon l'invention. Tout moyen de solidarisation connu par l'homme du métier peut être utilisé : épissure et/ou noeud ou fusion de la seconde extrémité de l'élément longiligne avec l'extrémité de la partie latérale d'ancrage 6.

Le dispositif d'attache 1 comprend des moyens de blocage 8 de ladite portion 2b dans le volume intérieur 3 dudit élément longiligne 2. Dans cet exemple précis, les moyens de blocage 8 comprennent un revêtement 9 dans au moins un polymère appliqué sur la surface extérieure et le pourtour de l'élément longiligne 2 sur la longueur (l1) séparant les orifices d'admission 4 et de sortie 5. De préférence, ledit au moins un polymère est choisi parmi le polyuréthane, le polydiméthylsiloxane (PDMS), l'acide polylactique de forme L ou D (PLLA ou PLDA), ou encore à base de polyamide, tel que le PA 66.

De préférence, la résistance de la liaison (i.e la résistance à rupture mesurée en daN) entre la seconde extrémité 2e de l'élément longiligne 2 et la partie latérale 6 doit être supérieure à la force de déblocage nécessaire pour la désactivation des moyens de blocage 8.

De préférence, la masse linéique du revêtement 9 par rapport à longueur dudit élément longiligne 2 est inférieure ou égale à 20 g/mètre dudit élément longiligne 2, de préférence inférieure ou égale à 15 g/mètre.

De préférence, ledit élément longiligne 2 est un élément textile tricoté ou tressé, et présente une masse linéaire inférieure à 10 g/mètre, de préférence inférieure à 5 g/mètre, et encore de préférence inférieure ou égale à 2 g/mètre.

De préférence, l'élément longiligne 2 est un élément textile tricoté ou tressé avec des fils multifilamentaires et/ou des fils monofilamentaires, de préférence des fils multifilamentaires. Lesdits fils sont de préférence choisis parmi le ou les polymères suivants, seuls ou en combinaison : polyéthylène téréphtalate, polypropylène, PLLA ou PLDA, polyéthylène.

Le titrage des fils est de préférence inférieur ou égal à 300 dtex, de préférence inférieur ou égal à 150 dtex.

L'élément longiligne 2 est de préférence une tresse obtenue par le tressage de six fils multifilamentaires, notamment en polyéthylène téréphtalate, ayant un titrage inférieur ou égal à 150 dtex.

La seconde variante du premier exemple de dispositif d'attache 10 représentée à la figure 2 diffère de la première variante 1 représentée à la figure 1 en ce que la portion 11b logée dans le volume intérieur 11a de l'élément longiligne 11 est inversée, de sorte qu'il est nécessaire d'exercer une traction sur la seconde extrémité 11e en liaison avec au moins une partie latérale d'ancrage 12 de l'implant 13 selon l'invention pour obtenir le coulissement de la portion 11b dans ledit volume intérieur 11a.

La première variante du second exemple de dispositif d'attache 14 représentée à la figure 3 diffère du premier exemple 1 de par les moyens de blocage 15 qu'il comporte. En effet, l'élément longiligne 16 ne comporte pas de revêtement disposé sur son pourtour entre les orifices d'admission 17 et de sortie 18 sur la longueur (I1) mais des première 19 et seconde 20 sphères dans au moins un polymère obturant respectivement les orifices d'admission 17 et de sortie 18. Les première 19 et seconde 20 sphères sont disposées respectivement autour des extrémités avant 16c et arrière 16d de la portion 16b de l'élément longiligne 16 logée dans son volume intérieur 16a, et agencées en sorte en sorte que la traction manuelle exercée sur la première extrémité 16f dudit élément longiligne 16 entraîne le passage en force de la première sphère 19 dans le volume intérieur 16a dudit élément longiligne 16 jusqu'à sa sortie par ledit orifice de sortie 18.

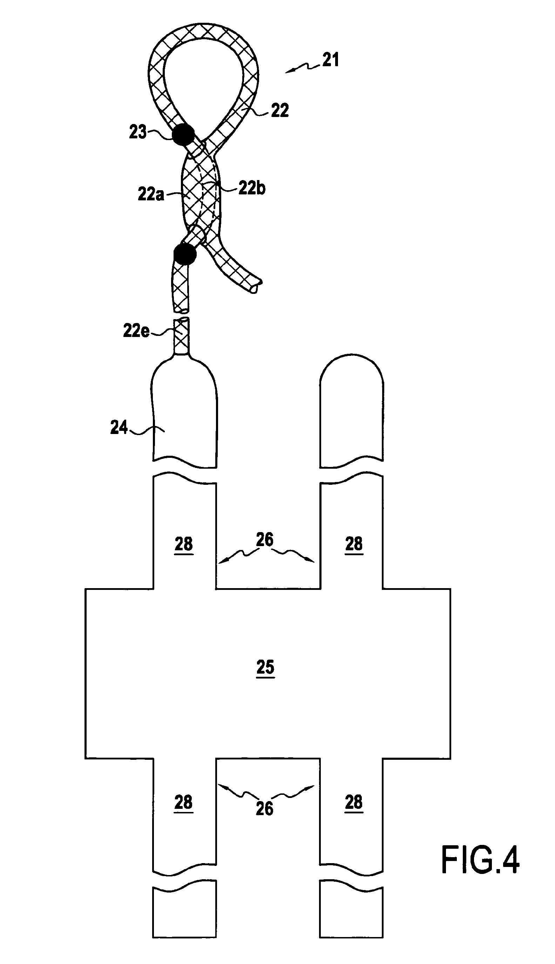

La seconde variante du dispositif d'attache 21 représentée à la figure 4 diffère de la première variante 14 représentée à la figure 3 en ce que la portion 22b de l'élément longiligne 22 logée dans son volume intérieur 22a est inversée, ce qui implique que le chirurgien doit exercer une traction sur la seconde extrémité 22e de l'élément longiligne 22 pour obtenir le passage en force de la première sphère 23 dans ledit volume intérieur 22a.

La figure 4 représente également dans son intégralité un implant 24 pour le traitement des prolapsus de la statique pelvienne comportant une partie centrale 25 supportante de laquelle se projettent deux paires 26,27 de parties latérales d'ancrage 28, dont au moins une est en liaison avec la seconde extrémité 22e de l'élément longiligne 22.

Le revêtement peut être éventuellement combiné avec une première sphère obturant l'orifice d'admission, et éventuellement encore avec une seconde sphère obturant l'orifice de sortie, du moment que la force de traction nécessaire pour faire passer la boucle de serrage d'une position inactive à une position active soit inférieure ou égale à 4 daN, de préférence inférieure ou égale à 2,5 daN. La force de traction est de préférence supérieure à 0,5 daN afin d'obtenir le passage, d'une position inactive à une position active pour la boucle de serrage, quasi-instantané, permettant d'assurer un serrage de la boucle de serrage fiable et reproductible.

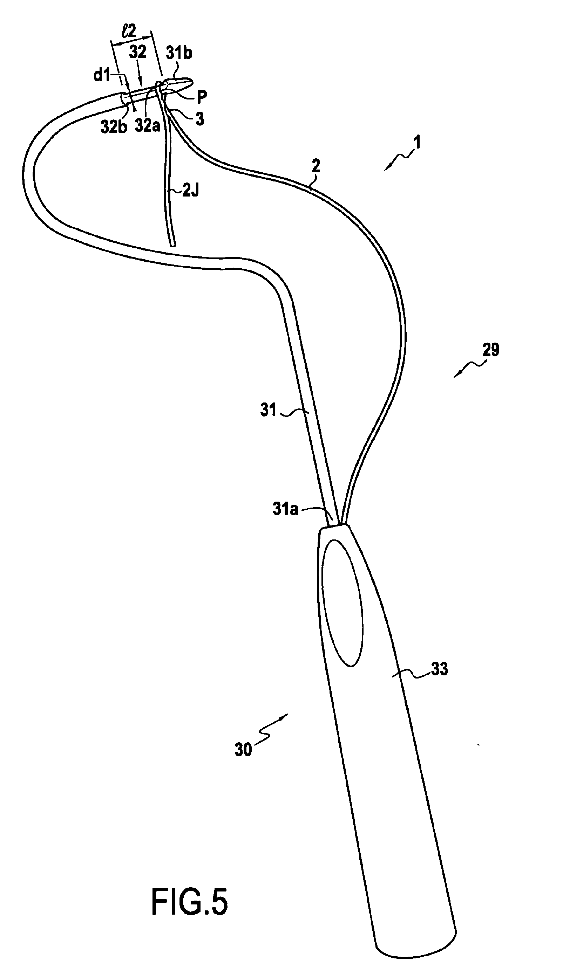

La figure 5 représente un kit 29 selon l'invention comprenant un ancillaire 30 comprenant une aiguille 31 pourvue d'une zone d'attache 32 apte à coopérer avec la boucle de serrage 3 du dispositif de serrage 1 représenté à la figure 1 dans sa position inactive.

L'ancillaire 30 comprend une poignée 33 ainsi qu'une aiguille 31 recourbée comportant des extrémités proximale 31a et distale 31b.

La zone d'attache 32 est disposée selon l'extrémité distale 31b de l'aiguille 31 et présente une longueur (12) ainsi que des butées avant 32a et arrière 32b pour la boucle de serrage 3 lorsque cette dernière est enfilée sur la zone d'attache 32.

Dans cet exemple précis, la zone d'attache 32 est de forme cylindrique et comporte un diamètre externe d1.

Bien évidemment, la circonférence en position inactive de la boucle de serrage 3 est inférieure ou égale à (P), de préférence inférieure ou égale à 5 cm, et encore de préférence inférieure ou égale à 3 cm, et dans tous les cas supérieure à n (pie) * d1.

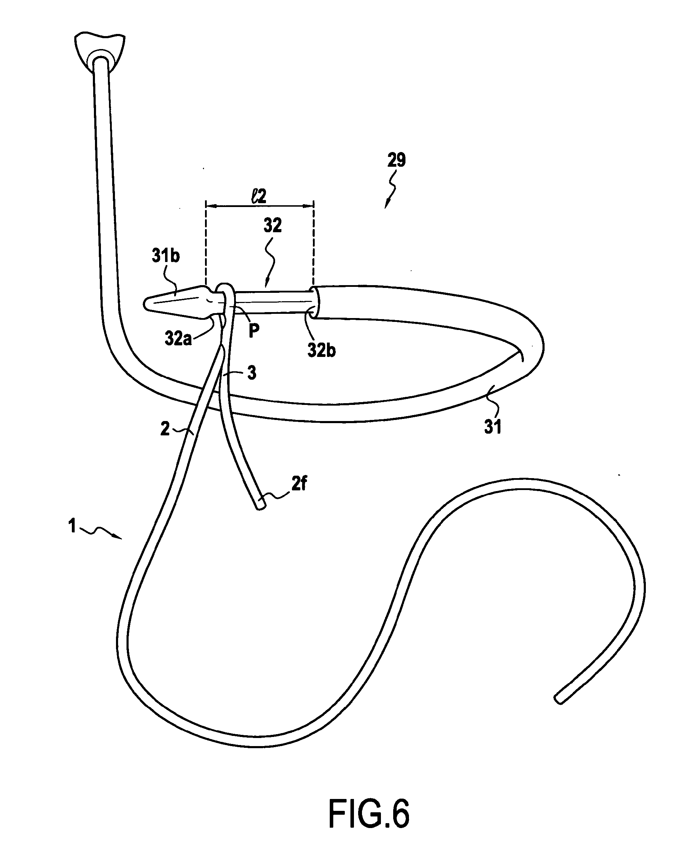

En fonctionnement, le chirurgien, après avoir passé l'aiguille 31 dans les tissus et/ou les trous trans-obturateurs et disposé la boucle de serrage 3 en position inactive sur la zone d'attache 32, exerce une traction d'un coup sec sur la première extrémité 2f de l'élément longiligne 2 afin de désactiver les moyens de blocage 8,9, pour dans ce cas obtenir la rupture du revêtement 9, et ainsi faire adopter à la boucle de serrage 3 sa circonférence en position active (p) telle que représentée à la figure 6. Le serrage est quasi-instantané, ce qui garantit son efficacité.

Par ailleurs, le serrage engendre une diminution de la longueur entre les orifices d'admission 4 et de sortie 5 du fait du coulissement dudit élément longiligne 2, ce qui améliore sa tenue dans le temps.

L'élément longiligne 2 étant très fin et dans un matériau textile, le frottement des fils entre-eux lors du coulissement contribue également à bloquer la boucle de serrage 3 autour de la zone de serrage 32 de l'aiguille 31.

Lorsque les moyens de blocage 8,9,19,20,23 comprennent une première sphère 19,23, cette dernière maintient rapprochés les orifices d'admission et de sortie en position active.

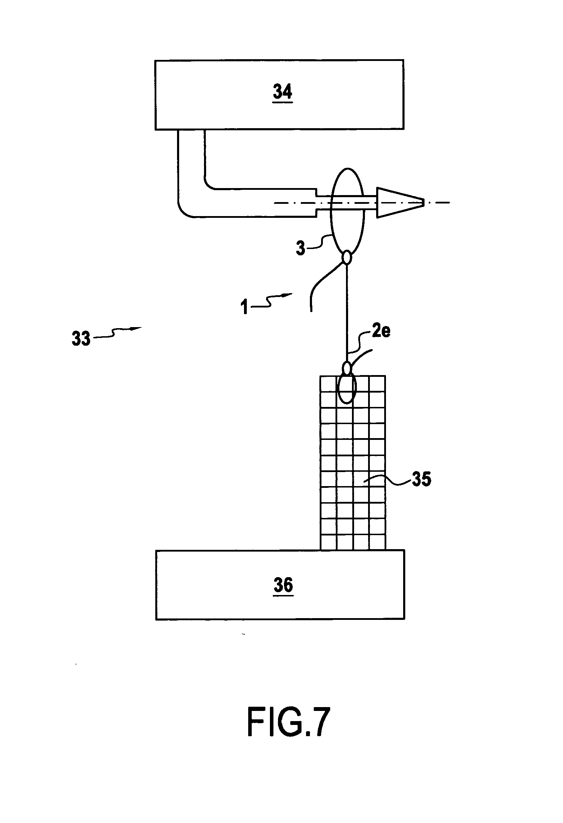

La figure 7 représente un banc de traction 33, par exemple de la marque LLOYD LR X, sur lequel la force nécessaire pour désactiver les moyens de blocage 8,9 du dispositif d'attache 1 représenté à la figure 1 est mesurée. La boucle de serrage 3 en position inactive est disposée autour d'un organe représentant l'extrémité distale 31b de l'aiguille 31 de l'ancillaire 30 selon l'invention, ledit organe étant maintenu entre des mors de serrage supérieurs 34. La seconde extrémité 2e du dispositif d'attache 1 est en liaison avec un textile 35 ayant une largeur de 1 cm et une hauteur de 10 cm. Le textile 35 est quant à lui pincé entre des mors de serrage inférieurs 36. La hauteur séparant les mors inférieurs 36 des mors supérieurs 34 est de 20 cm. La vitesse de traction est à titre d'exemple de 100 mm / minute.

## Revendications

1. Dispositif d'attache implantable (1, 10, 14, 21) comprenant un élément longiligne tubulaire souple et creux (2, 11, 16, 22) délimitant un volume intérieur (2a, 11a, 16a, 22a) comportant une boucle de serrage (3), de circonférence (P) en position inactive, formée par la disposition d'une portion (2b, 11b, 16b, 22b) dudit élément tubulaire (2, 11, 16, 22) dans ledit volume intérieur (2a, 11a, 16a, 22a) entre des orifices d'admission (4, 17) et de sortie (5,18) distant d'une longueur (I1), ladite portion (2b, 11b, 16b, 22b) ayant des extrémités avant (2c, 16c) et arrière (2d, 16d), et en ce que le dispositif (1, 10, 14, 21) comprend des moyens de blocage (8, 15) de ladite portion (2b, 11b, 16b, 22b) dans le volume intérieur (2a, 11a, 16a, 22a) dudit élément longiligne (2, 11, 16, 22), lesdits moyens de blocage (8, 15) étant désactivés sous l'effet d'une traction manuelle exercée sur la première (2f, 16f) ou seconde (2e, 11e, 22e) extrémité dudit élément longiligne (2, 11, 16, 22), ladite traction permettant le coulissement dudit élément longiligne (2, 11, 16, 22) entre lesdits orifices (4, 5, 17, 18) et corrélativement la formation d'une boucle de serrage (3) de circonférence (p) en position active, (p) étant inférieur à (P).

2. Dispositif d'attache (1, 10) selon la revendication 1, **caractérisé en ce que** les moyens de blocage (8) comprennent un revêtement (9) dans au moins un polymère appliqué sur la surface extérieure et le pourtour de l'élément longiligne (2, 11) sur la longueur (l1) séparant les orifices d'admission (4) et de sortie (5).

3. Dispositif d'attache (1, 10) selon la revendication 2, **caractérisé en ce que** la masse du revêtement (9) est inférieure ou égale à 20 g par mètre dudit élément tubulaire souple (2, 11).

4. Dispositif (14, 21) selon l'une quelconque des revendications 1 à 3, **caractérisé en ce que** les moyens de blocage (15) comprennent au moins une première sphère (19, 23) dans au moins un polymère obturant l'orifice d'admission (17), ladite sphère (19, 23) étant disposée autour de l'extrémité avant (16c) de ladite portion (16b, 22b), et agencée en sorte que la traction manuelle exercée sur la première (16f) ou la seconde (22e) extrémité dudit élément longiligne (16, 22) entraîne le passage en force de la dite première sphère (19, 23) dans le volume intérieur (16a, 22a) dudit élément longiligne (16, 22) jusqu'à sa sortie par ledit orifice de sortie (18).

5. Dispositif (14, 21) selon la revendication 4, **caractérisé en ce que** les moyens de blocage (15) comprennent une seconde sphère (20) dans au moins un polymère obturant l'orifice de sortie (18), ladite sphère (20) étant disposée autour de l'extrémité arrière (16d) de ladite portion (16b, 22b).

6. Dispositif d'attache (1, 10, 14, 21) selon l'une quelconque des revendications 1 à 5, **caractérisé en ce que** ledit matériau polymère est choisi seul ou en combinaison parmi les polymères suivants : polydiméthysiloxane (PDMS), polyuréthane, polymère d'acide lactique de forme L ou D (PLLA ou PLDA), polyamide (PA 66 notamment).

7. Dispositif (1, 10, 14, 21) selon l'une quelconque des revendications 1 à 6, **caractérisé en ce que** la boucle de serrage (3) présente une circonférence (P) en position inactive inférieure à 5 cm, de préférence inférieure à 3 cm.

8. Dispositif (1, 10, 14, 21) selon l'une quelconque des revendications 1 à 7, **caractérisé en ce que** la longueur (l1) séparant les orifices d'admission (17) et de sortie (18) est inférieure ou égale à 3 cm, de préférence inférieure ou égale à 1 cm.

9. Dispositif (1, 10, 14, 21) selon l'une quelconque des revendications 1 à 8, **caractérisé en ce que** l'élément longiligne (2, 11, 16, 22) est un élément textile, en particulier une tresse ou un tricot, comprenant un ou plusieurs fil(s) multifilamentaire(s) et/ou un ou plusieurs fil(s) monofilamentaire(s).

10. Dispositif (1, 10, 14, 21) selon l'une quelconque des revendications 1 à 9, caractérisé en que l'élément longiligne (2, 11, 16, 22) a une masse linéique inférieure ou égale à 25 g/mètre, de préférence inférieure ou égale à 15 g/mètre.

11. Dispositif (1, 10, 14, 21) selon l'une quelconque des revendications 1 à 10, **caractérisé en ce que** la traction exercée sur la première (2f, 16f) ou la seconde extrémité (2e, 11e, 22e) permettant de désactiver les moyens de blocage (8, 15) est inférieure ou égale à 4 daN, de préférence inférieure ou égale à 2,5 daN.

12. Implant (7, 13, 24) pour le traitement des prolapsus de la statique pelvienne, notamment pour la cure de l'hystérocèle, de la cystocèle ou de la rectocèle, configuré en sorte de présenter une partie centrale supportante (25) et au moins deux parties latérales d'ancrage (6, 12, 26, 27, 28) disposées de part et d'autre de ladite partie centrale (25) **caractérisé en ce qu'**il comprend un dispositif d'attache (1, 10, 14, 21) selon l'une des revendications 1 à 11, et **en ce que** la seconde extrémité (22e) dudit élément longiligne (22) est solidarisée ou apte à être solidarisée à l'une des parties latérale d'ancrage.

13. Kit (29) comprenant un dispositif d'attache (1, 10, 14, 21) selon l'une des revendications 1 à 11, **caractérisé en ce qu'**il comprend un ancillaire (30) comprenant une aiguille (31) pourvue selon son extrémité distale (31b) d'une zone d'attache (32) apte à coopérer avec la boucle de serrage (3) dudit dispositif d'attache (1, 10, 14, 21).

14. Kit (29) selon la revendication 13, **caractérisé en ce que** la zone d'attache (32) présente une longueur (12) et comporte des butées avant (32a) et arrière (32b) aptes à bloquer dans ladite zone d'attache (32) la boucle de serrage (3) dudit élément longiligne (2, 11, 16, 22) lorsque la boucle de serrage (3) est en position active (p).

## Patentansprüche

1. Implantierbare Befestigungsvorrichtung (1, 10, 14, 21), umfassend ein biegsames und hohles röhrenförmiges längliches Element (2, 11, 16, 22), das ein Innenvolumen (2a, 11a, 16a, 22a) begrenzt, umfassend eine Klemmschlaufe (3) in Umfangsrichtung (P) in inaktiver Position, die durch die Anordnung eines Abschnitts (2b, 11b, 16b, 22b) des röhrenförmigen Elements (2, 11, 16, 22) in dem Innenvolumen (2a, 11a, 16a, 22a) zwischen Eintritts- (4, 17) und Austrittsöffnungen (5, 18), die um eine Länge (l1) entfernt sind, gebildet ist, wobei der Abschnitt (2b, 11b, 16b, 22b) vordere Enden (2c, 16c) und hintere Enden (2d, 16d) aufweist, und dass die Vorrichtung (1, 10, 14, 21) Mittel (8, 15) zur Feststellung des Abschnitts (2b, 11b, 16b, 22b) in dem Innenvolumen (2a, 11a, 16a, 22a) des länglichen Elements (2, 11, 16, 22) umfasst, wobei die Feststellungsmittel (8, 15) unter der Wirkung eines manuellen Zugs, der auf das erste (2f, 16f) oder zweite (2e, 11e, 22e) Ende des länglichen Elements (2, 11, 16, 22) ausgeübt wird, deaktiviert werden, wobei der Zug das Gleiten des länglichen Elements (2, 11, 16, 22) zwischen den Öffnungen (4, 5, 17, 18) und in Korrelation die Bildung einer Klemmschlaufe (3) in Umfangsrichtung (p) in aktiver Position ermöglicht, wobei (p) kleiner als (P) ist.

2. Befestigungsvorrichtung (1, 10) nach Anspruch 1, **dadurch gekennzeichnet, dass** die Feststellungsmittel (8) eine Verkleidung (9) aus mindestens einem Polymer umfassen, das auf die Außenfläche und den Umfang des länglichen Elements (2, 11) auf die Länge (l1) zwischen den Eintritts- (4) und Austrittsöffnungen (5) aufgebracht wird.

3. Befestigungsvorrichtung (1, 10) nach Anspruch 2, **dadurch gekennzeichnet, dass** die Masse der Verkleidung (9) kleiner oder gleich 20 g pro Meter des biegsamen röhrenförmigen Elements (2, 11) ist.

4. Vorrichtung (14, 21) nach einem der Ansprüche 1 bis 3, **dadurch gekennzeichnet, dass** die Feststellungsmittel (15) mindestens eine erste Kugel (19, 23) aus mindestens einem Polymer umfassen, die die Eintrittsöffnung (17) verschließt, wobei die Kugel (19, 23) um das vordere Ende (16c) des Abschnitts (16b, 22b) angeordnet und derart vorgesehen ist, dass der manuelle Zug, der auf das erste (16f) oder das zweite (22e) Ende des länglichen Elements (16, 22) ausgeübt wird, zur festen Durchführung der ersten Kugel (19, 23) durch das Innenvolumen (16a, 22a) des länglichen Elements (16, 22) bis zu ihrem Austritt durch die Austrittsöffnung (18) führt.

5. Vorrichtung (14, 21) nach Anspruch 4, **dadurch gekennzeichnet, dass** die Feststellungsmittel (15) eine zweite Kugel (20) aus mindestens einem Polymer, die die Austrittsöffnung (18) verschließt, umfassen, wobei die Kugel um das hintere Ende (16d) des Abschnitts (16b, 22b) angeordnet ist.

6. Befestigungsvorrichtung (1, 10, 14, 21) nach einem der Ansprüche 1 bis 5, **dadurch gekennzeichnet, dass** das Polymermaterial nur oder in Kombination unter den folgenden Polymeren ausgewählt ist: Polydimethylsiloxan (PDMS), Polyurethan, Milchsäurepolymer der Form L oder D (PLLA oder PLDA), Polyamid (PA 66 insbesondere).

7. Vorrichtung (1, 10, 14, 21) nach einem der Ansprüche 1 bis 6, **dadurch gekennzeichnet, dass** die Klemmschlaufe (3) einen Umfang (P) in inaktiver Position von weniger als 5 cm, vorzugsweise weniger als 3 cm, aufweist.

8. Vorrichtung (1, 10, 14, 21) nach einem der Ansprüche 1 bis 7, **dadurch gekennzeichnet, dass** die Länge (l1) zwischen den Eintritts- (17) und Austrittsöffnungen (18) kleiner oder gleich 3 cm, vorzugsweise kleiner oder gleich 1 cm, ist.

9. Vorrichtung (1, 10, 14, 21) nach einem der Ansprüche 1 bis 8, **dadurch gekennzeichnet, dass** das längliche Element (2, 11, 16, 22) ein textiles Element, insbesondere ein Geflecht oder ein Gewebe, ist, umfassend einen oder mehrere Multifilamentfäden und/oder einen oder mehrere Monofilamentfäden.

10. Vorrichtung (1, 10, 14, 21) nach einem der Ansprüche 1 bis 9, **dadurch gekennzeichnet, dass** das längliche Element (2, 11, 16, 22) eine längenbezogene Masse kleiner oder gleich 25 g/Meter, vorzugsweise kleiner oder gleich 15 g/Meter, hat.

11. Vorrichtung (1, 10, 14, 21) nach einem der Ansprüche 1 bis 10, **dadurch gekennzeichnet, dass** der auf das erste (2f, 16f) oder zweite Ende (2e, 11e, 22e) ausgeübte Zug, der es ermöglicht, die Feststellungsmittel (8, 15) zu deaktivieren, kleiner oder gleich 4 daN, vorzugsweise kleiner oder gleich 2,5 daN, ist.

12. Implantat (7, 13, 24) zur Behandlung von Vorfällen im Beckenbereich, insbesondere zur Behandlung der Hysterozele, der Zystozele oder der Rektozele, das derart ausgeführt ist, dass es einen tragenden zentralen Teil (25) und mindestens zwei seitliche Verankerungsteile (6, 12, 26, 27, 28) umfasst, die beiderseits des zentralen Teils (25) angeordnet sind, **dadurch gekennzeichnet, dass** es eine Befestigungsvorrichtung (1, 10, 14, 21) nach einem der Ansprüche 1 bis 11 umfasst, und dass das zweite Ende (22e) des länglichen Elements (22) mit einem der seitlichen Verankerungsteile verbunden ist oder geeignet ist, verbunden zu werden.

13. Bausatz (29), umfassend eine Befestigungsvorrichtung (1, 10, 14, 21) nach einem der Ansprüche 1 bis 11, **dadurch gekennzeichnet, dass** es ein Hilfsinstrument (30) umfasst, das eine Nadel (31) aufweist, die entlang ihres distalen Endes (31 b) mit einer Befestigungszone (32) versehen ist, die geeignet ist, mit der Klemmschlaufe (3) der Befestigungsvorrichtung (1, 10, 14, 21) zusammenzuwirken.

14. Bausatz (29) nach Anspruch 13, **dadurch gekennzeichnet, dass** die Befestigungszone (32) eine Länge (l2) aufweist und vordere (32a) und hintere Anschläge (32b) umfasst, die geeignet sind, in der Befestigungszone (32) die Klemmschlaufe (3) des länglichen Elements (2, 11, 16, 22) festzustellen, wenn sich die Klemmschlaufe (3) in aktiver Position (p) befindet.

## Claims

1. An implantable attachment device (1, 10, 14, 21) comprising an elongate tubular element (2, 11, 16, 22) which is flexible and hollow delimiting an internal volume (2a, 11a, 16a, 22a) comprising a tightening loop (3), of circumference (P) in the inactive position, formed by the arrangement of a portion (2b, 11b, 16b, 22b) of said tubular element (2, 11, 16, 22) in said internal volume (2a, 11a, 16a, 22a) between intake (4, 17) and outlet (5, 18) openings distant by a length (l1), said portion (2b, 11 b, 16b, 22b) having front (2c, 16c) and rear (2d, 16d) ends, and in that the device (1, 10, 14, 21) comprises blocking means (8, 15) of said portion (2b, 11b, 16b, 22b) in the internal volume (2a, 11a, 16a, 22a) of said elongate element (2, 11, 16, 22), said blocking means (8, 15) being deactivated under the effect of manual traction exerted on the first (2f, 16f) or second (2e, 11e, 22e) end of said elongate element (2, 11, 16, 22), said traction enabling the sliding of said elongate element (2, 11, 16, 22) between said orifices (4, 5, 17, 18) and correlatively the formation of a tightening loop (3) of circumference (p) in the active position, (p) being less than (P).

2. The attachment device (1, 10) according to Claim 1, **characterised in that** the blocking means (8) comprise a coating (9) in at least one polymer applied to the external surface and the periphery of the elongate element (2, 11) over the length (l1) separating the intake (4) and outlet (5) openings.

3. The attachment device (1, 10) according to Claim 2, **characterised in that** the mass of the coating (9) is less than or equal to 20 g per meter of said tubular flexible element (2, 11).

4. The device (14, 21) according to any one of Claims 1 to 3, **characterised in that** the blocking means (15) comprise at least one first sphere (19, 23) in at least one polymer blocking the intake opening (17), said sphere (19, 23) being arranged about the front end (16c) of said portion (16b, 22b), and arranged such that manual traction exerted on the first (16f) or the second (22e) end of said elongate element (16, 22) causes the forced passage of said first sphere (19, 23) in the internal volume (16a, 22a) of said elongate element (16, 22) until it exits via said discharge outlet (18).

5. The device (14, 21) according to Claim 4, **characterised in that** the blocking means (15) comprise a second sphere (20) in at least one polymer blocking the discharge outlet (18), said sphere (20) being arranged about the end rear (16d) of said portion (16b, 22b).

6. The attachment device (1, 10, 14, 21) according to any one of Claims 1 to 5, **characterised in that** said polymer material is selected alone or in combination from the following polymers: polydimethysiloxane (PDMS), polyurethane, lactic acid polymer of L or D shape (PLLA or PLDA), polyamide (PA 66 especially).

7. The device (1, 10, 14, 21) according to any one of Claims 1 to 6, **characterised in that** the tightening loop (3) has a circumference (P) in the inactive position of less than 5 cm, preferably less than 3 cm.

8. The device (1, 10, 14, 21) according to any one of Claims 1 to 7, **characterised in that** the length (l1) separating the intake (17) and outlet (18) openings is less than or equal to 3 cm, preferably less than or equal to 1 cm.

9. The device (1, 10, 14, 21) according to any one of Claims 1 to 8, **characterised in that** the elongate element (2, 11, 16, 22) is a textile element, in particular a braid or a knit, comprising one or more multifilament threads and/or one or more monofilament threads.

10. The device (1, 10, 14, 21) according to any one of Claims 1 to 9, **characterised in that** the elongate element (2, 11, 16, 22) has a mass per unit less than or equal to 25 g/meter, preferably less than or equal to 15 g/meter.

11. The device (1, 10, 14, 21) according to any one of Claims 1 to 10, **characterised in that** the traction exerted on the first (2f, 16f) or the second end (2e, 11e, 22e) to deactivate the blocking means (8, 15) is less than or equal to 4 daN, preferably less than or equal to 2.5 daN.

12. An implant (7, 13, 24) for the treatment of prolapse of the pelvic floor, especially for curing hysterocele, cystocele or rectocele, configured so as to present a central supporting part (25) and at least two lateral anchoring parts (6, 12, 26, 27, 28) arranged on either side of said central part (25), **characterised in that** it comprises an attachment device (1, 10, 14, 21) according to any one of Claims 1 to 11, and **in that** the second end (22e) of said elongate element (22) is attached to or capable of being attached to one of the lateral anchoring parts.

13. A kit (29) comprising an attachment device (1, 10, 14, 21) according to any one of Claims 1 to 11, **characterised in that** it comprises un instrument (30) comprising a needle (31) provided at its distal end (31 b) with an attachment zone (32) capable of cooperating with the tightening loop (3) of said attachment device (1, 10, 14, 21).

14. The kit (29) according to Claim 13, **characterised in that** the attachment zone (32) has a length (l2) and comprises front (32a) and rear (32b) stops capable of blocking in said attachment zone (32) the tightening loop (3) of said elongate element (2, 11, 16, 22) when the tightening loop (3) is in the active position (p).
